# EUROPÄISCHE PATENTSCHRIFT

(11) **EP 4 601 589 B1**
(45) Veröffentlichungstag und Bekanntmachung des Hinweises auf die Patenterteilung: **19.08.2026**
(21) Anmeldenummer: 22802536.7
(22) Anmeldetag: 10.10.2022
(51) Int. Cl.: A61F 2/70, G05G 5/05

(54) **SENSOREINRICHTUNG**
SENSOR DEVICE
DISPOSITIF DE CAPTEURS

(43) Veröffentlichungstag der Anmeldung: 20.08.2025
(73) Patentinhaber: Otto Bock Healthcare Products GmbH, 1110 Wien (AT)
(72) Erfinder: DJORDJEVIC, Danijel, 1110 Wien (AT); BOHLAND, Andreas, 1110 Wien (AT); ASSELN, Malte, 1110 Wien (AT)
(74) Vertreter: Gramm, Lins & Partner Patent- und Rechtsanwälte PartGmbB
(86) Internationale Anmeldenummer: PCT/EP2022/078152
(87) Internationale Veröffentlichungsnummer: WO 2024/078694

(56) Entgegenhaltungen:
- EP-B1- 2 341 410

## Beschreibung

Die Erfindung betrifft eine Sensoreinrichtung, die wenigstens zwei Sensoren und einen Grundkörper mit einem Oberteil und einem Unterteil und wenigstens einem zwischen dem Oberteil und dem Unterteil angeordneten elastischen Element aufweist.

Derartige Sensoreinrichtungen sind aus dem Stand der Technik, beispielsweise der EP 2 341 410 A1, bekannt. Die WO 2020/041491 A1 beschreibt beispielsweise eine solche Anordnung, die in einer orthopädietechnischen Einrichtung, beispielsweise einem Verbindungselement zum Verbinden zweier Prothesenbauteile angeordnet ist. Die Einrichtung verfügt über ein Oberteil, auf dem sich ein herkömmlicher Pyramidenadapter zum Verbinden mit einem anderen Prothesenbauteil befindet. Das Unterteil ist als plattenförmiges Element ausgebildet und über einen zentral angeordneten Steg mit dem Oberteil verbunden. Dadurch kann das Oberteil relativ zum Unterteil verkippt werden, wenn der zentrale Steg verformt wird. An den jeweils äußeren Rändern der Oberteile und Unterteile sind Sensoren angeordnet, die einen Abstand zwischen dem Oberteil und dem Unterteil messen können. Damit lässt sich ein Winkel zwischen dem Oberteil und dem Unterteil ermitteln. Dieser Winkel wird hervorgerufen, wenn auf gegenüberliegenden Seiten des Steges unterschiedliche Kräfte herrschen. Nachteilig ist, dass durch die nur geringe Auslenkung sehr genaue Sensoren benötigt werden und die Endanschläge der Auslenkung früh erreicht werden.

Aus der WO 2021/040998 A1 ist eine ähnliche Einrichtung bekannt. Sie weist einen Rahmen auf, der in sich verformbar ist, wenn Kräfte auf ihn wirken. Auch diese Einrichtung ist für die Verwendung in orthopädietechnischen Einrichtungen vorgesehen. Sie verfügt über eine Basis, die den Pyramidenadapter zum Verbinden mit anderen Prothesenbauteilen trägt und die das Unterteil bildet. Diese Basis weist eine sich von medial nach lateral erstreckende Erhebung auf, auf der der Rahmen, der das Oberteil bildet, aufliegt. Anterior und posterior von dieser Erhebung ist zwischen dem Oberteil und dem Unterteil ein kleiner Spalt vorhanden, der es erlaubt, dass sich das Oberteil unter der Einwirkung von Kräften bewegt, wobei diese Bewegung eine Verformung des Oberteiles bedingt. In dieser Ausführung können nur Kräfte ermittelt werden, die zu einer Verformung des Oberteiles führen.

Aus der US 8,746,080 B2 ist eine Verbindungseinrichtung für orthopädietechnische Einrichtungen bekannt, die einen Pyramidenadapter mit einem Hohlraum aufweist. Dadurch kommt es bei großen Belastungen zu Verformungen des Adapters und damit des Hohlraumes, die durch Abstandssensoren bestimmt werden können. Auch hier müssen die Kräfte zu einer Verformung des Oberteiles, das durch den oberen Teil des Adapters gebildet wird, führen, um gemessen werden zu können.

Der Erfindung liegt daher die Aufgabe zugrunde, die Nachteile aus dem Stand der Technik zu beheben oder zumindest zu reduzieren.

Die Erfindung löst die gestellte Aufgabe durch eine Sensoreinrichtung gemäß Anspruch 1.

Anders als bei den Ausgestaltungen aus dem Stand der Technik kann bei der erfindungsgemäßen Ausgestaltung das Oberteil auch relativ zu dem Unterteil entlang der Vorzugsrichtung verschoben werden, vorzugsweise ohne dass es dabei zu einer Verformung des Oberteils kommt. Die wenigstens zwei Sensoren sind eingerichtet, einen Abstand zwischen dem Oberteil und dem Unterteil zu bestimmen, wobei dies an zwei unterschiedlichen Orten geschieht. In vorteilhaften Ausgestaltungen sind mehr als zwei, vorzugsweise mehr als drei, besonders bevorzugt mehr als vier Sensoren vorhanden, die jeweils einen Abstand zwischen dem Oberteil und dem Unterteil bestimmen. Die Positionen, denen dies geschieht, sind vorzugsweise so angeordnet, dass sie nicht in einer geraden Linie liegen.

Die Sensoren müssen dabei nicht in der Lage sein, einen Abstand zwischen dem Oberteil und dem Unterteil quantitativ zu bestimmen, auch wenn dies eine bevorzugte Ausgestaltung ist. Für die Funktion der Erfindung ist es ausreichend, wenn jeder Sensor in der Lage ist, zu bestimmen, ob an seiner Position ein Abstand zwischen dem Oberteil und dem Unterteil vorhanden ist oder nicht. Daher kann ein solcher Sensor beispielsweise als Kontaktsensor oder Drucksensor ausgebildet sein oder eine andere Art von Sensor sein, mit der erkannt werden kann, ob das Oberteil am Unterteil anliegt.

Vorzugsweise ist die Größe der Abstände zwischen dem Oberteil und dem Unterteil für jeden einzelnen Sensor im unbelasteten Zustand bekannt. Dies entspricht einer Nullstellung. Die einzelnen Abstände müssen im unbelasteten Zustand nicht für alle Sensoren gleich sein. Es ist jedoch von Vorteil, wenn sie es sind. Wirkt nun eine Kraft auf das Oberteil und/oder das Unterteil, die zu einer Bewegung des Oberteils relativ zum Unterteil führt, verändern sich wenigstens einige, in der Regel jedoch alle Abstände zwischen Oberteil und Unterteil, die von jeweils einem Sensor gemessen werden. Ändern sich alle Abstände gleich, bedeutet dies, dass das Oberteil und das Unterteil aufeinander zu bewegt wurden, ohne dass sich die Orientierung des Oberteils relativ zum Unterteil verändert hat. Es handelt sich folglich um eine Verschiebung entlang der Vorzugsrichtung.

Verändern sich jedoch die Abstände zwischen dem Oberteil und dem Unterteil in unterschiedlicher Weise an unterschiedlichen Positionen, verändert sich auch die Orientierung des Oberteils relativ zum Unterteil. Es handelt sich folglich um eine Verkippung um die Kippachse. Natürlich sind auch Überlagerungen der beiden Bewegungen möglich, etwa wenn alle Abstände zwischen Oberteil und Unterteil, die von Sensoren detektiert werden, kleiner werden, jedoch um unterschiedliche Beträge reduziert werden.

In der erfindungsgemäßen Ausgestaltung der Sensoreinrichtung wird anders als im Stand der Technik nicht das Oberteil oder das Unterteil, sondern ein vorzugsweise dazwischen angeordnetes elastisches Element verformt. Vorzugsweise ist mehr als ein elastisches Element, beispielsweise zwei, drei oder vier elastische Elemente vorhanden, die bei einer Bewegung des Oberteils relativ zum Unterteil verformt werden. Dabei ist es nicht notwendig, dass bei jeder Bewegung alle elastischen Elemente verformt werden. Die elastischen Elemente sind dabei so angeordnet, dass die Stärke und die Art der Verformung, die bei den elastischen Elementen stattgefunden hat, aus dem Messergebnis der Sensoren, die den Abstand zwischen Oberteil und Unterteil bestimmen, ermittelt werden können. Die Federkonstanten, also der Zusammenhang zwischen Verformung eines elastischen Elementes und der dazu führenden Kraft, sind vorzugsweise für alle Federelemente bekannt. Dieser Zusammenhang wird auch dann Federkonstante genannt, wenn der Zusammenhang kein linearer Zusammenhang ist. Aus der Kenntnis all dieser Größen lässt sich dann die Größe und die Richtung der auf die Sensoreinrichtung wirkenden Kraft bestimmen.

Durch die Verwendung wenigstens eines elastischen Elementes werden größere Verschiebungen und/oder Verkippungen des Oberteils relativ zu dem Unterteil über dern zu messenden Kraft- und /oder Momentenbereich ermöglicht, als das bei Ausführungen aus dem Stand der Technik der Fall ist. Dadurch können auch Sensoren mit geringerer Auflösung und Messgenauigkeit verwendet werden, die in der Regel kostengünstiger sind. Eine ausreichende mechanische Stabilität der Sensoreinrichtung wird bevorzugt durch Endanschläge sichergestellt, bei deren Erreichen große Kräfte und/oder Momente vom Oberteil auf das Unterteil übertragen werden können, ohne dass das wenigstens eine elastische Element zu starken Belastungen ausgesetzt wird.

**In** einer bevorzugten Ausgestaltung stellt das wenigstens eine elastische Element einer Verkippung des Oberteils relativ zum Unterteil in eine erste Kipprichtung einen anderen Widerstand als einer Verkippung in eine zweite Kipprichtung entgegen. Dies ist beispielsweise dann von Vorteil, wenn die erwarteten Kräfte und/oder die daraus resultierenden und erzeugten Drehmomente in unterschiedliche Kipprichtungen unterschiedlich groß sind.

Vorzugsweise ist das Oberteil relativ zu dem Unterteil entlang einer Symmetrieebene des Grundkörpers, bevorzugt einer Symmetrieebene der Sensoreinrichtung verschiebbar. Vorzugsweise wird die Sensoreinrichtung in einer orthopädietechnischen Einrichtung, beispielsweise einer Orthese oder einer Prothese, um die dort auftretenden Kräfte und Drehmomente zu bestimmen. Die Symmetrieebene des Grundkörpers bevorzugte Symmetrieebene der Sensoreinrichtung ist dann vorzugsweise so angeordnet, dass sie einer Frontalebene oder einer Sagittalebene des Trägers der orthopädietechnischen Einrichtung entspricht. Die Vorzugsrichtung, entlang derer das Oberteil relativ zum Unterteil verschiebbar ist und die in der Symmetrieebene liegt, erstreckt sich dann vorzugsweise von proximal nach distal.

Vorzugsweise liegt die Kippachse in der Symmetrieebene. Die Symmetrieebene wird dann bevorzugt durch die Kippachse und die Vorzugsrichtung aufgespannt.

Vorteilhafterweise liegen die wenigstens zwei Sensoren auf gegenüberliegenden Seiten der Symmetrieebene und weisen vorzugsweise den gleichen Abstand zu dieser Symmetrieebene auf. Auf diese Weise ist die Berechnung der wirkenden Kräfte aus den Abstandsmessungen der Sensoren auf besonders einfache Weise möglich. Für den Fall, dass die Kippachse nicht in der Symmetrieebene liegt, sondern beispielsweise zu ihr verkippt ausgebildet ist, ist es von Vorteil, wenn die Sensoren den gleichen Abstand von der Kippachse aufweisen.

Vorteilhafterweise ist das Oberteil mit dem Unterteil durch wenigstens einen Verbindungssteg verbunden. Das wenigstens eine elastische Element weist dann Scharniere, vorzugsweise Filmscharniere, auf, durch die der Verbindungssteg mit dem Oberteil und dem Unterteil verbunden ist. Kommt es nun zu einer Bewegung des Oberteils relativ zum Unterteil verändern sich die Positionen der Scharniere. Um die Verbindungsstege weiterhin mit dem Oberteil und dem Unterteil verbunden zu halten, muss sich also auch das Scharnier bewegen und einen anderen Winkel einnehmen. Dieser Bewegung des Scharniers setzt das Scharnier, insbesondere als Filmscharnier, eine elastische Kraft entgegen. Die Bewegung des Scharniers entspricht folglich der Verformung des elastischen Elementes. Je nach Ausgestaltung der Scharniere, insbesondere der Filmscharniere kann die zum Verformen der elastischen Elemente nötige Kraft unterschiedlich groß ausgebildet sein.

In einer bevorzugten Ausgestaltung befindet sich wenigstens ein Verbindungssteg auf jeder Seite der Symmetrieebene und/oder der Kippachse. Jedes der Scharniere weist zwei relativ zueinander verdrehbare oder verschwenkbare Elemente auf, die als Laschen oder Lappen bezeichnet werden. Jeweils eine dieser Laschen eines jeden Scharniere ist mit dem Verbindungssteg verbunden, während die andere der beiden Laschen mit dem Oberteil oder dem Unterteil verbunden ist. Ist das Scharnier als Filmscharnier ausgebildet, sind die beiden Laschen vorzugsweise einstückig miteinander ausgebildet. In einer besonders bevorzugten Ausgestaltung sind das Oberteil, das Unterteil, die Verbindungsstege und die Scharniere einstückig ausgebildet.

Bevorzugt weist die Sensoreinrichtung wenigstens zwei Anschläge auf, durch die eine Verkippung des Oberteils relativ zu dem Unterteil in jeweils eine Richtung begrenzt wird. Damit wird eine Überbeanspruchung und Überlastung der elastischen Elemente verhindert. Dies bedeutet jedoch, dass Drehmomente, die für eine Verkippung des Oberteils relativ zum Unterteil in die jeweilige Richtung sorgen, nur bis zu einem Maximalwert gemessen oder ermittelt werden können.

Vorzugsweise sind die Anschläge am Unterteil angeordnet und das Oberteil kommt mit diesen Anschlägen in Kontakt, sobald es ausreichend weit in die jeweilige Richtung relativ zum Unterteil verschwenkt wurde. Am Oberteil kann dafür ein Kontaktelement, beispielsweise in Form einer gehärteten Platte, angeordnet sein. Selbst verständlich kann auch die umgekehrte Anordnung also der Anschlag am Oberteil und des Kontaktelement am Unterteil verwendet werden. In einer besonders bevorzugten Ausgestaltung befindet sich einer der wenigstens zwei Sensoren im Kontaktelement oder in einem der Anschläge.

Vorzugsweise verfügt die Sensoreinrichtung über wenigstens einen Zusatzanschlag, durch den die Verschiebung des Oberteils relativ zu dem Unterteil begrenzt wird. Der Zusatzanschlag befindet sich vorzugsweise zwischen den beiden Anschlägen. Besonders bevorzugt befindet sich der Zusatzanschlag in der Vorzugsrichtung. Höchst bevorzugt befindet sich der Zusatzanschlag in der Symmetrieebene des Grundkörpers, vorzugsweise in der Symmetrieebene der Sensoreinrichtung. Prinzipiell wird auch die Verschiebung des Oberteils relativ zum Unterteil durch die beiden Anschläge begrenzt, die auch die Verkippung begrenzen. Wenn einer der Anschläge an dem jeweiligen Kontaktelement anliegt, ist eine Verkippung in die jeweilige Richtung nicht weiter möglich. Liegen beide Anschläge an ihrem jeweiligen Kontaktelement an, ist eine Verschiebung des Oberteils relativ zum Unterteil nicht weiter möglich. Es ist dann allerdings nicht möglich, zu unterscheiden, ob zusätzlich zu der großen Kraft auch ein gegebenenfalls kleineres Drehmoment auf das Oberteil oder die Sensoreinrichtung wirkt. Dies wird mit einem Zusatzanschlag erreicht. Der Zusatzanschlag ist aber vorzugsweise so ausgebildet, dass wenn das Kontaktelement des Zusatzanschlages an diesem anliegt, eine Verkippung des Oberteils relativ zum Unterteil weiterhin möglich ist.

Besonders bevorzugt verfügt der Zusatzanschlag über eine Abrollkontur, sodass das Oberteil auf dem Unterteil oder umgekehrt abrollen kann. Dadurch wird eine weitere Verkippung erleichtert selbst wenn eine weitere Verschiebung durch den Zusatzanschlag bereits ausgeschlossen ist. Selbst verständlich kann auch der Zusatzanschlag am Oberteil oder am Unterteil und das jeweilige Kontaktelement am jeweils anderen Bauteil des Grundkörpers angeordnet sein.

In einer bevorzugten Ausgestaltung verfügt die Sensoreinrichtung über einen Zusatzsensor, der in der Symmetrieebene angeordnet ist und eingerichtet ist, den Abstand zwischen dem Oberteil und dem Unterteil zu bestimmen. Dieser Zusatzsensor befindet sich vorzugsweise im Zusatzanschlag oder dessen Kontaktelement.

Vorteilhafterweise beinhalten die wenigstens zwei Sensoren und/oder der Zusatzsensor wenigstens einen Hall-Sensor und/oder wenigstens einen optischen Sensor. Mit den wenigstens zwei Sensoren und/oder dem Zusatzsensor ist es möglich, berührungslos den Abstand zu messen.

In einer bevorzugten Ausgestaltung ist am Oberteil oder am Unterteil des Grundkörpers ein Permanentmagnet angeordnet. Am jeweils anderen Teil sind wenigstens zwei Hall-Sensoren derart positioniert, dass sie sich im Magnetfeld des Permanentmagneten befinden. Wird nun eine Kraft auf das Oberteil des Grundkörpers ausgeübt, folgt daraus eine Verschiebung und/oder eine Verkippung des Oberteils, die eine Bewegung des Permanentmagneten relativ zu den beiden Hall-Sensoren zur Folge hat. Vorzugsweise sind die beiden Sensoren derart angeordnet, dass eine reine Verschiebung des Oberteils relativ zum Unterteil zur Folge hat, dass sich der Abstand zwischen dem Permanentmagneten und den beiden Hall-Sensoren in gleichem Maße ändert. Die Sensoren detektieren dann eine Veränderung des Magnetfeldes, wobei diese Veränderung ebenfalls für beide Hall-Sensoren gleich groß ist. So kann detektiert werden, dass es sich ausschließlich um eine Verschiebung des Oberteils relativ zum Unterteil, also eine Longitudinal-Bewegung, handelt. Führt die auf das Oberteil und/oder das Unterteil wirkende Kraft zusätzlich zu einer Verkippung des Oberteils relativ zum Unterteil, hat dies zur Folge, dass sich der Abstand zwischen dem Permanentmagneten und den beiden Hall-Sensoren nicht gleichmäßig, sondern für beide Sensoren unterschiedlich ändert. Dadurch wird auch das von den beiden Sensoren detektiert Magnetfeld unterschiedlich stark verändert. Auf diese Weise kann der Kippwinkel um eine bestimmte Achse ermittelt werden.

Diese Ausgestaltung der wenigstens zwei Sensoren hat den Vorteil, dass sie unabhängig vom Aufbau des Grundkörpers direkt eine Verkippung und/oder Verschiebung des Oberteils relativ zu dem Unterteil misst. Ein elastisches Element ist dabei von Vorteil, jedoch nicht notwendig. Eine Sensoreinrichtung, die wenigstens zwei Hall-Sensoren und einen Grundkörper mit einem Oberteil und einem Unterteil aufweist, wobei die Hall-Sensoren am Oberteil und ein Permanentmagnet am Unterteil oder die Hall-Sensoren am Unterteil und der Permanentmagnet am Oberteil angeordnet sind, und wobei sich die Sensoreinrichtung dadurch auszeichnet, dass das Oberteil relativ zu dem Unterteil aus einer Null-Lage heraus entlang der Vorzugsrichtung verschiebbar und um eine senkrecht zu der Vorzugsrichtung stehende Kippachse kippbar ist, wobei die wenigstens zwei Hall-Sensoren eingerichtet sind, jeweils einen Abstand zwischen dem Oberteil und dem Unterteil zu bestimmen, so dass eine Verschiebung und/oder Verkippung des Oberteils relativ zu dem Unterteil aus der Null-Lage heraus bestimmbar ist, stellt daher eine eigene Erfindung dar. Diese eigene Erfindung ist mit allen anderen Merkmalen, die vorliegend beschrieben sind und die sich nicht auf das wenigstens eine elastische Element beziehen, kombinierbar.

Besonders bevorzugt sind die wenigstens zwei Hall-Sensoren symmetrisch zur Symmetrieebene des Grundkörpers, vorzugsweise symmetrisch zur Symmetrieebene der gesamten Sensoreinrichtung, positioniert. In einer besonders bevorzugten Ausgestaltung sind wenigstens drei, besonders bevorzugt wenigstens vier Hall-Sensoren vorhanden, die nicht entlang einer einzigen Geradenlinie positioniert sind. Auf diese Weise lässt sich eine Verkippung um jede Achse detektieren, die unterschiedlich starke Veränderungen der detektieren Magnetfelder bei den verwendeten Hall-Sensoren zur Folge hat.

Vorzugsweise beinhalten die wenigstens zwei Sensoren und/oder der Zusatzsensor wenigstens einen Kontaktsensor, einen Drucksensor und/oder einen kapazitiven Sensor. Ein solcher Sensor ist bevorzugt derart angeordnet und eingerichtet, dass er in der Lage ist, festzustellen, ob sich zwischen dem Oberteil und dem Unterteil ein Abstand befindet oder ob sich das Oberteil und das Unterteil berühren.

Mithilfe der beigefügten Figuren werden nachfolgend einige Ausführungsbeispiele der vorliegenden Erfindung näher erläutert. Es zeigen
Figuren 1 bis 3 schematische Darstellungen von Funktionsweisen, die in der vorliegenden Erfindung verwirklicht sind,
Figuren 4 bis 7 schematische Darstellungen eines Grundkörpers unter verschiedenen Krafteinwirkungen und
Figuren 8 bis 10 die schematische Seitenansicht, Schnittdarstellung und Sensorpositionierung eines Grundkörpers.

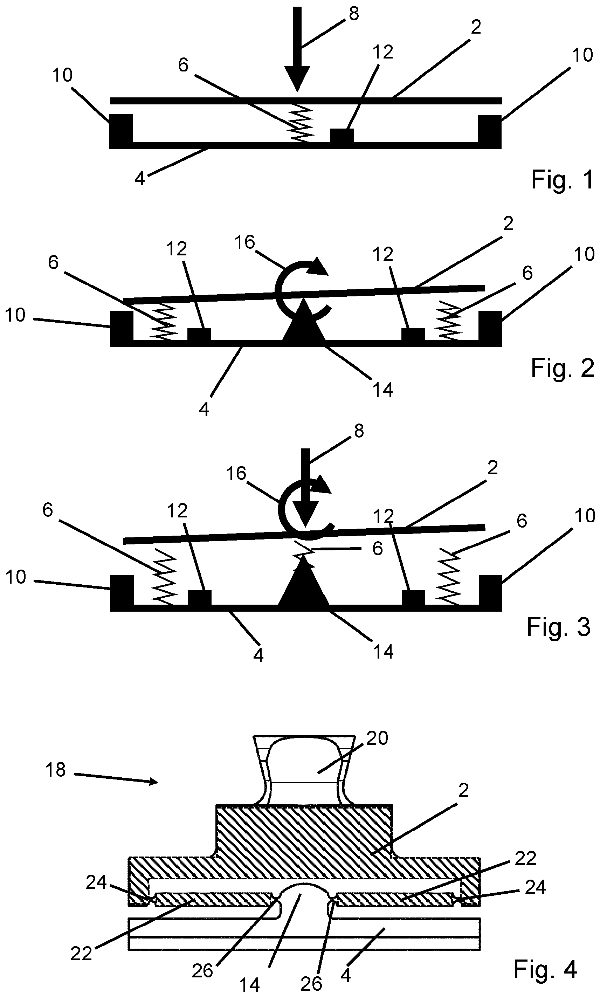

Figur 1 zeigt schematisch eine Funktion, die in einer Sensoreinrichtung gemäß einem Ausführungsbeispiel der vorliegenden Erfindung realisiert ist. Es ist schematisch ein Oberteil 2 sowie ein Unterteil 4 eines Grundkörpers gezeigt. Zwischen beiden befindet sich ein elastisches Element 6, das in der gezeigten schematische Darstellung als Feder ausgebildet ist. Wird nun eine Kraft 8, die durch den Pfeil dargestellt ist, in die dem Pfeil entsprechende Richtung, wird das elastische Element 6 komprimiert. Durch die Ausgestaltung und Richtung der Kraft 8 kommt es zu einer Verschiebung des Oberteils 2 relativ zum Unterteil 4 entlang der Richtung der wirkenden Kraft 8. Figur 1 illustriert die Situation, in der ausschließlich eine Verschiebung zwischen dem Oberteil 2 und dem Unterteil 4 stattfindet.

In dieser Situation sind zwei Anschläge 10 vorhanden, durch die die maximal mögliche Verschiebung begrenzt wird. Dies ist dann der Fall, wenn das Oberteil 2 an die Anschläge 10 anschlägt. Zwischen dem Oberteil 2 und dem Unterteil 4 ist ein Sensor 12 dargestellt, durch den der Abstand zwischen dem Oberteil 2 und dem Unterteil 2 messbar ist.

Figur 2 illustriert eine Situation, in der ausschließlich eine Verkippung des Oberteils 2 relativ zum Unterteil 4 möglich ist. Anstelle des einen elastischen Elementes 6, das in Figur 1 in der Mitte angeordnet ist, sind in Figur 2 zwei elastische Element 6 dargestellt. In der Mitte befindet sich ein Zusatzanschlag 14 auf dem das Oberteil aufliegt. Diese Darstellung wurde deswegen gewählt, um lediglich eine Verkippung, nicht jedoch eine Verschiebung des Oberteils 2 relativ zum Unterteil 4 zu erlauben. An den beiden Seiten sind wieder die Anschläge 10 vorhanden. Anders als in Figur 1, bei der durch eine Verschiebung des Oberteils 2 dieses nur gemeinsame mit beiden Anschlägen 10 in Kontakt kommen konnte, kann in Figur 2 das Oberteil 2 nur mit einem der beiden Anschläge 10 in Kontakt kommen. Dies ist dann möglich, wenn ein Drehmoment 16 wirkt, das durch den gebogenen Pfeil dargestellt wird. Dabei soll der Pfeil lediglich darstellen, dass ein Drehmoment wirkt. Die Richtung des Pfeiles soll nicht die Richtung des Drehmoments festlegen.

Die in Figur 2 gezeigte Anordnung verfügt über zwei Sensoren 12, die jeweils eingerichtet sind, an ihrer Position den Abstand zwischen Oberteil 2 und Unterteil 4 zu bestimmen. Über die unterschiedlichen Abstände zwischen Oberteil 2 und Unterteil 4 sowie die Position der Sensoren 12 lässt sich der Winkel des Oberteils 2 relativ zum Unterteil 4 bestimmen und damit aus den bekannten Kraftverläufen der elastischen Elemente 6 das wirkende Drehmoment bestimmen. Man erkennt in Figur 2 bereits, dass ein elastisches Element 6 auch außer Eingriff mit dem Oberteil 2 kommen kann, wenn dies beispielsweise durch eine durch ein Drehmoment 16 hervorgerufene Verschwenkung geschieht. In Figur 2 ist dies beim rechten der beiden elastischen Elemente 6 der Fall.

In Figur 3 ist die Kombination der beiden Situation aus Figur 1 und Figur 2 dargestellt. Dies entspricht funktional einer Sensoreinrichtung gemäß einem Ausführungsbeispiel der vorliegenden Erfindung. Zwischen dem Oberteil 2 und dem Unterteil 4 sind in diesem Fall drei elastische Elemente 6 positioniert. Diese werden unterschiedlich stark komprimiert, wenn eine Kraft 8 und/oder ein Drehmoment 16 wirkt. Im gezeigten Ausführungsbeispiel sind die beiden seitlichen elastischen Elemente 6 noch nicht in Kontakt mit dem Oberteil 2. Dies ist jedoch nicht notwendigerweise der Fall. Ausgestaltungen, bei denen die seitlichen elastischen Elemente 6 immer mit dem Oberteil 2 in Kontakt sind, können in besonderen Situation ebenfalls von Vorteil sein.

Die in Figur 3 gezeigte Ausführungsform weist die beiden Anschläge 10, die beiden Sensoren 12 sowie den Zusatzanschlag 14 auf.

Figur 4 zeigt eine Ausgestaltung eines Grundkörpers 18 für eine Sensoreinrichtung gemäß einem Ausführungsbeispiel der vorliegenden Erfindung. Der Grundkörper verfügt über einen Pyramidenadapter 20, der Teil des Oberteils 2 ist. Das Unterteil 4 verfügt über den Zusatzanschlag 14. Zwischen dem Oberteil 2 und dem Zusatzanschlag 14 des Unterteil 4 sind in Figur 4 zwei Verbindungsstege 22 dargestellt, deren in Figur 4 äußeres Ende über ein Filmscharnier 24 mit dem Oberteil 2 verbunden ist. Das in Figur 4 innere Ende der Verbindungsstege 22 ist über ein weiteres Filmscharnier 26 mit dem Zusatzanschlag 14 des Unterteil 4 verbunden.

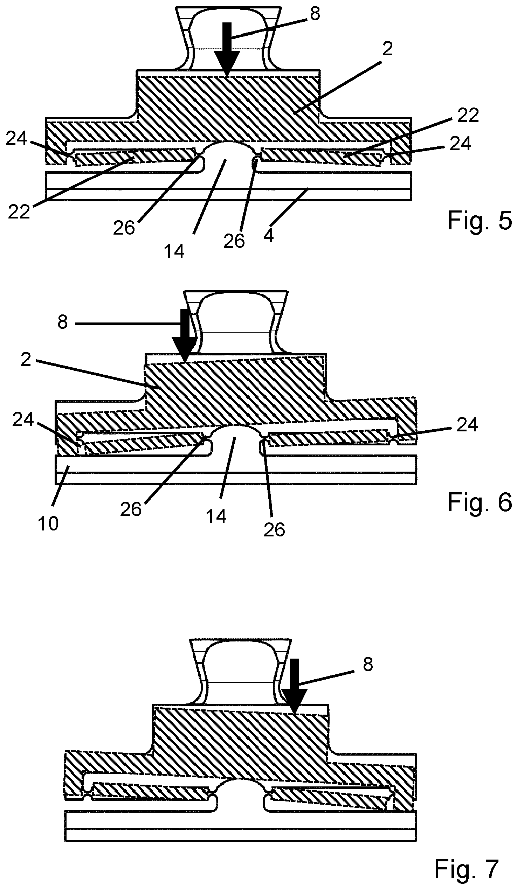

Figur 5 zeigt die Ausgestaltung aus Figur 4 unter der Einwirkung einer Kraft 8. Man erkennt, dass das Oberteil 2 relativ zum Unterteil 4 nach unten verschoben wurde, sodass die Verbindungsstege 22 nicht mehr parallel zueinander und nicht mehr parallel zum Unterteil 4 verlaufen. Um diese Position zu erreichen, sind die Filmscharniere 24, 26 elastische verformt worden. Sie bilden in diesem Fall die elastischen Elemente 6. Die Verschiebung des Oberteils 2 relativ zum Unterteil 4, die durch die Kraft 8 hervorgerufen wurde, endete in dem Moment, in dem das Oberteil 2 an dem Zusatzanschlag 14 anlag.

Figur 6 zeigt die Situation aus Figur 5, wobei die Kraft 8 anders als in Figur 5 nicht mehr zentral, also entlang der Symmetrieachse des Grundkörpers 18 sondern zu dieser versetzt wirkt. Dadurch entsteht auch ein Drehmoment 16, das jedoch nicht grafisch dargestellt ist. Das Oberteil 2 ist durch die wirkende Kraft aus der in Figur 4 gezeigten Ruheposition sowohl verschoben als auch verschwenkt worden. Das Oberteil liegt an dem Zusatzanschlag 14 und an dem links angeordneten Anschlag 10 an. Auch in diesem Beispiel sind die Filmscharniere 24, 26 verformt worden und bilden die elastischen Elemente. In Figur 7 ist die entsprechend umgekehrte Situation gezeigt, in der die Kraft 8 nicht wie in Figur 6 links von der Symmetrieachse, sondern rechts von der Symmetrieachse des Grundkörpers 18 bewirkt. Man erkennt insbesondere in den Figuren 5 und 6, dass die obere Kontaktfläche des Zusatzanschlages 14 gebogen und als Abrollfläche ausgebildet ist, sodass das Oberteil 2 auch dann besonders leicht verkippt werden kann, wenn es bereits mit dem Zusatzanschlag 14 in Kontakt kommt.

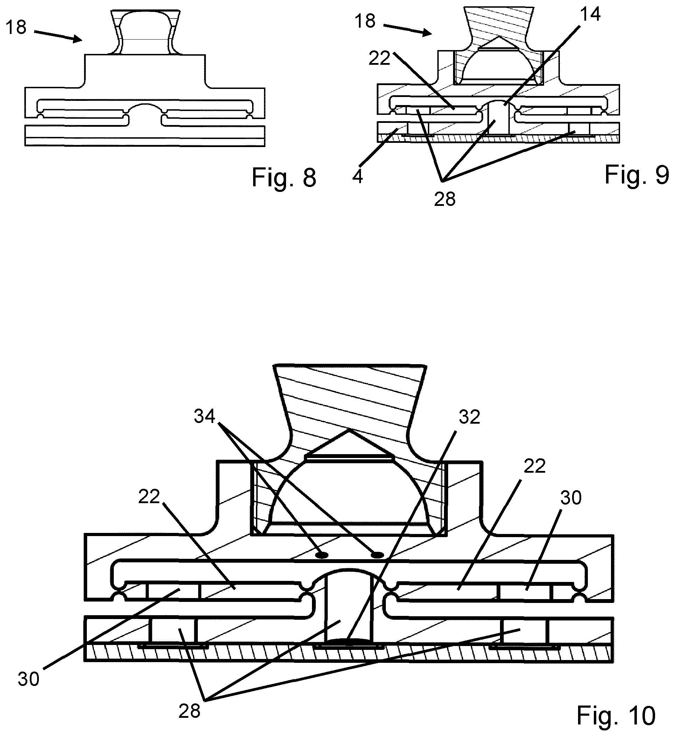

Figur 8 zeigt eine Seitenansicht eines Grundkörpers 18, wie er bereits in Figur 4 dargestellt wurde. Figur 9 entgegen zeigt eine Schnittdarstellung durch den Grundkörper 18 aus Figur 8. Man erkennt Messkanäle 28, die im Unterteil 4 beginnen. Ein mittlerer Messkanal 28 verläuft durch den Zusatzanschlag 14 und ist nach oben Richtung Oberteil 2 offen ausgebildet. Die beiden seitlichen Messkanäle 28 beginnen ebenfalls im Unterteil 4 und erstrecken sich durch Öffnungen in den Verbindungssteg 22 hindurch.

Figur 10 zeigt die vergrößerte Darstellung aus Figur 9. Man erkennt die Messkanäle 28 und die Öffnungen 30 in den Verbindungssteg 22. Am Boden des mittleren Messkanals 28 befindet sich einem Permanentmagnet 32, dessen Magnetfeld sich insbesondere durch den mittleren Messkanal 28 erstreckt. An der dem Unterteil 4 zugewandten Seite des Oberteils 2 sind zwei Hall-Sensoren 34 angeordnet. Diese messen das Magnetfeld des Permanentmagneten 32 sehr genau und können so Änderungen im Abstand zwischen dem Oberteil 2 und dem Unterteil 4 sehr genau detektieren. Zusätzlich können sie aufgrund ihrer Anordnung feststellen, wenn die Änderung an den beiden jeweiligen Positionen der Hall-Sensoren 34 unterschiedlich stark ausfällt. Daraus lässt sich eine Verkippung berechnen. Zusätzlich oder alternativ dazu können auch in den seitlichen Messkanälen 28 Sensoren angeordnet sein.

### Bezugszeichen-Liste

- 2: Oberteil
- 4: Unterteil
- 6: elastisches Element
- 8: Kraft
- 10: Anschlag
- 12: Sensor
- 14: Zusatzanschlag
- 16: Drehmoment
- 18: Grundkörper
- 20: Pyramidenadapter
- 22: Verbindungssteg
- 24: Filmscharnier
- 26: Filmscharnier
- 28: Messkanal
- 30: Öffnung
- 32: Permanentmagnet
- 34: Hall-Sensor

## Patentansprüche

1. Sensoreinrichtung zum Bestimmen von Kräften und Drehmomenten in einer orthopädietechnischen Einrichtung, die
- wenigstens zwei Sensoren (12) und
- einen Grundköper (18) mit einem Oberteil (2) und einem Unterteil (4) und wenigstens einem zwischen dem Oberteil (2) und dem Unterteil (4) angeordneten elastischen Element (6) aufweist,
wobei das Oberteil (2) relativ zu dem Unterteil (4) aus einer Null-Lage heraus um eine senkrecht zu einer Vorzugsrichtung stehende Kippachse kippbar ist, wobei wenigstens ein elastisches Element (6) verformt wird und die wenigstens zwei Sensoren (12) eingerichtet sind, jeweils einen Abstand zwischen dem Oberteil (2) und dem Unterteil (4) zu bestimmen, **dadurch gekennzeichnet, dass** das Oberteil (2) relativ zu dem Unterteil (4) entlang der Vorzugsrichtung verschiebbar und die Sensoren (12) den Abstand so bestimmen, dass eine Verschiebung und Verkippung des Oberteils (2) relativ zu dem Unterteil (4) aus der Null-Lage heraus bestimmbar ist.

2. Sensoreinrichtung nach Anspruch 1, **dadurch gekennzeichnet, dass** das wenigstens eine elastische Element (6) einer Verkippung des Oberteils (2) relativ zu dem Unterteil (4) in eine erste Kipprichtung einen anderen Widerstand als einer Verkippung in eine zweite Kipprichtung entgegenstellt.

3. Sensoreinrichtung nach Anspruch 1 oder 2, **dadurch gekennzeichnet, dass** das Oberteil (2) relativ zu dem Unterteil (4) entlang einer Symmetrieebene des Grundkörpers (18), bevorzugt einer Symmetrieebene der Sensoreinrichtung verschiebbar ist.

4. Sensoreinrichtung nach Anspruch 3, **dadurch gekennzeichnet, dass** die Kippachse in der Symmetrieebene liegt.

5. Sensoreinrichtung nach Anspruch 3 oder 4, **dadurch gekennzeichnet, dass** die beiden Sensoren (12) auf gegenüberliegenden Seiten der Symmetrieebene liegen und vorzugsweise den gleichen Abstand zu der Symmetrieebene aufweisen.

6. Sensoreinrichtung nach einem der vorstehenden Ansprüche, **dadurch gekennzeichnet, dass** das Oberteil (2) mit dem Unterteil (4) durch wenigstens einen Verbindungssteg (22) verbunden ist, und das wenigstens eine elastische Element (6) Scharniere, insbesondere Filmscharniere (24,26) aufweist, durch die der Verbindungssteg (22) mit dem Oberteil (2) und dem Unterteil (4) verbunden ist.

7. Sensoreinrichtung nach einem der vorstehenden Ansprüche, **dadurch gekennzeichnet, dass** die Sensoranordnung wenigstens zwei Anschläge (10) aufweist, durch die eine Verkippung des Oberteils (2) relativ zu dem Unterteil (4) in jeweils eine Richtung begrenzt wird.

8. Sensoreinrichtung nach einem der vorstehenden Ansprüche, **dadurch gekennzeichnet, dass** die Sensoranordnung wenigstens einen Zusatzanschlag (14) aufweist, durch den die Verschiebung des Oberteils (2) relativ zu dem Unterteil (4) begrenzt wird.

9. Sensoreinrichtung nach Anspruch 8, **dadurch gekennzeichnet, dass** der Zusatzanschlag (14) eine Abrollkontur aufweist, auf der das Oberteil (2) auf dem Unterteil (4) abrollen kann.

10. Sensoreinrichtung nach einem der Ansprüche 3 bis 9, **dadurch gekennzeichnet, dass** die Sensoreinrichtung einen Zusatzsensor aufweist, der in der Symmetrieebene angeordnet ist und eingerichtet ist, den Abstand zwischen dem Oberteil (2) und dem Unterteil (4) zu bestimmen.

11. Sensoreinrichtung nach einem der vorstehenden Ansprüche, **dadurch gekennzeichnet, dass** die wenigstens zwei Sensoren (12) und/oder der Zusatzsensor wenigstens einen Hall-Sensor (34) und/oder wenigstens einen optischen Sensor beinhalten.

12. Sensoreinrichtung nach einem der vorstehenden Ansprüche, **dadurch gekennzeichnet, dass** die wenigstens zwei Sensoren (12) und/oder der Zusatzsensor wenigstens einen Kontaktsensor, einen Drucksensor und/oder einen kapazitiven Sensor beinhalten.

## Claims

1. A sensor device to determine forces and toques in an orthopedic device, comprising
- at least two sensors (12) and
- a main body (18) with an upper part (2) and a lower part (4) and at least one elastic element (6) arranged between the upper part (2) and the lower part (4),
wherein the upper part (2) can be tilted from a zero position relative to the lower part (4) about a tilt axis perpendicular to a preferred direction, wherein at least one elastic element (6) is deformed and the at least two sensors (12) are each configured to determine a distance between the upper part (2) and the lower part (4), **characterized in that** the upper part (2) can be displaced relative to the lower part (4) along the preferred direction and that the sensors (12) determine the distance such, that a displacement and tilt of the upper part (2) relative to the lower part (4) from the zero position can be determined.

2. The sensor device according to claim 1, **characterized in that** the at least one elastic element (6) opposes a tilt of the upper part (2) relative to the lower part (4) in a first tilt direction with a different resistance to a tilt in a second tilt direction.

3. The sensor device according to claim 1 or 2, **characterized in that** the upper part (2) can be displaced relative to the lower part (4) along a plane of symmetry of the main body (18), preferably a plane of symmetry of the sensor device.

4. The sensor device according to claim 3, **characterized in that** the tilt axis lies in the plane of symmetry.

5. The sensor device according to claim 3 or 4, **characterized in that** the two sensors (12) are on opposite sides of the plane of symmetry and are preferably at the same distance from said plane of symmetry.

6. The sensor device according to one of the preceding claims, **characterized in that** the upper part (2) is connected to the lower part (4) by at least one connecting bar (22) and the at least one elastic element (6) comprises hinges, especially film hinges (24,26), by means of which the connecting bar (22) is connected to the upper part (2) and the lower part (4).

7. The sensor device according to one of the preceding claims, **characterized in that** the sensor arrangement has at least two end stops (10) each of which limits a tilt of the upper part (2) relative to the lower part (4) in one direction.

8. The sensor device according to one of the preceding claims, **characterized in that** the sensor arrangement has at least one additional end stop (14) by means of which the displacement of the upper part (2) relative to the lower part (4) is limited.

9. The sensor device according to claim 8, **characterized in that** the additional end stop (14) has a rolling contour on which the upper part (2) can roll on the lower part (4).

10. The sensor device according to one of the claims 3 to 9, **characterized in that** the sensor device has an additional sensor that is arranged in the plane of symmetry and configured to determine the distance between the upper part (2) and the lower part (4).

11. The sensor device according to one of the preceding claims, **characterized in that** the at least two sensors (12) and/or the additional sensor include at least one Hall sensor (34) and/or at least one optical sensor.

12. The sensor device according to one of the preceding claims, **characterized in that** the at least two sensors (12) and/or the additional sensor include at least one contact sensor, a pressure sensor and/or at least one capacitive sensor.

## Revendications

1. Dispositif capteur destiné à déterminer les forces et les couples de rotation dans un dispositif orthopédique, comprenant
- au moins deux capteurs (12) et
- un corps de base (18) comportant une partie supérieure (2) et une partie inférieure (4), ainsi qu'au moins un élément élastique (6) disposé entre la partie supérieure (2) et la partie inférieure (4),
la partie supérieure (2) étant apte à basculer par rapport à la partie inférieure (4) à partir d'une position zéro autour d'un axe de basculement perpendiculaire à une direction préférentielle, au moins un élément élastique (6) étant déformé, et lesdits au moins deux capteurs (12) étant agencés pour déterminer chacun une distance entre la partie supérieure (2) et la partie inférieure (4),
**caractérisé en ce que** la partie supérieure (2) est apte à coulisser par rapport à la partie inférieure (4) le long de la direction préférentielle, et les capteurs (12) déterminent la distance de manière à permettre de déterminer un coulissement et un basculement de la partie supérieure (2) par rapport à la partie inférieure (4) à partir de la position zéro.

2. Dispositif capteur selon la revendication 1,
**caractérisé en ce que** ledit au moins un élément élastique (6) oppose une résistance à un basculement de la partie supérieure (2) par rapport à la partie inférieure (4) dans une première direction de basculement, ladite résistance étant différente d'une résistance opposée à un basculement dans une deuxième direction de basculement.

3. Dispositif capteur selon la revendication 1 ou 2,
**caractérisé en ce que** la partie supérieure (2) est apte à coulisser par rapport à la partie inférieure (4) le long d'un plan de symétrie du corps de base (18), de préférence le long d'un plan de symétrie du dispositif capteur.

4. Dispositif capteur selon la revendication 3,
**caractérisé en ce que** l'axe de basculement se trouve dans le plan de symétrie.

5. Dispositif capteur selon la revendication 3 ou 4,
**caractérisé en ce que** les deux capteurs (12) sont situés de part et d'autre du plan de symétrie et sont de préférence à égale distance du plan de symétrie.

6. Dispositif capteur selon l'une des revendications précédentes, **caractérisé en ce que** la partie supérieure (2) est reliée à la partie inférieure (4) par au moins une barrette de liaison (22), et ledit au moins un élément élastique (6) comporte des charnières, en particulier des charnières à film (24, 26), par lesquelles la barrette de liaison (22) est reliée à la partie supérieure (2) et à la partie inférieure (4).

7. Dispositif capteur selon l'une des revendications précédentes, **caractérisé en ce que** le dispositif capteur comporte au moins deux butées (10) qui limitent chacune le basculement de la partie supérieure (2) par rapport à la partie inférieure (4) dans un sens respectif.

8. Dispositif capteur selon l'une des revendications précédentes, **caractérisé en ce que** le dispositif capteur comporte au moins une butée supplémentaire (14) qui limite le coulissement de la partie supérieure (2) par rapport à la partie inférieure (4).

9. Dispositif capteur selon la revendication 8,
**caractérisé en ce que** la butée supplémentaire (14) présente un profil de roulement sur lequel la partie supérieure (2) peut rouler sur la partie inférieure (4).

10. Dispositif capteur selon l'une des revendications 3 à 9,
**caractérisé en ce que** le dispositif capteur comprend un capteur supplémentaire qui est disposé dans le plan de symétrie et qui est conçu pour déterminer la distance entre la partie supérieure (2) et la partie inférieure (4).

11. Dispositif capteur selon l'une des revendications précédentes, **caractérisé en ce que** lesdits au moins deux capteurs (12) et/ou le capteur supplémentaire incluent au moins un capteur à effet Hall (34) et/ou au moins un capteur optique.

12. Dispositif capteur selon l'une des revendications précédentes, **caractérisé en ce que** lesdits au moins deux capteurs (12) et/ou le capteur supplémentaire incluent au moins un capteur de contact, un capteur de pression et/ou un capteur capacitif.
